Europäisches Patentamt

(19) European Patent Office

Office européen des brevets

(11) Publication number: **0 251 232**
**B1**

# EUROPEAN PATENT SPECIFICATION

(45) Date of publication of the patent specification:
**14.11.90**

(51) Int. Cl.⁵: **C07C 321/24**, C07C 319/28

(21) Application number: **87109192.2**

(22) Date of filing: **26.06.87**

(54) Decontamination of (hydrocarbylthio) aromatic amines.

(30) Priority: **26.06.86 US 878813**

(43) Date of publication of application:
**07.01.88 Bulletin 88/1**

(45) Publication of the grant of the patent:
**14.11.90 Bulletin 90/46**

(84) Designated Contracting States:
**DE FR GB IT**

(56) References cited:
**EP-A- 0 169 101**
**EP-A- 0 172 612**
**US-A- 4 324 920**

(73) Proprietor: **ETHYL CORPORATION, Ethyl Tower 451 Florida Boulevard, Baton Rouge Louisiana 70801(US)**

(72) Inventor: **Davis, Robert Lynn, 1343 Dahlia, Baton Rouge LA. 70808(US)**

(74) Representative: **Sandmair, Kurt, Dr. et al, Patentanwälte Schwabe, Sandmair, Marx Stuntzstrasse 16, D-8000 München 80(DE)**

**Description**

DECONTAMINATION OF (HYDROCARBYLTHIO)AROMATIC AMINES

This invention relates to (hydrocarbylthio)aromatic amines and more particularly to a process for decontaminating them.

As disclosed in U. S. Patent 4,594,453 (Ranken et al.), it is known that (hydrocarbylthio)aromatic amines can be prepared by reacting an aromatic amine with a hydrocarbyl disulfide in the presence of a catalytic amount of a Lewis acid. The Lewis acid contaminant can be removed by diluting the product with an organic liquid such as toluene, washing with an aqueous acid or base, and then removing the diluent and water. However, this decontamination technique is bothersome, uneconomical, and apt to be particularly unsatisfactory when the amine is to be used in an application in which even small amounts of water cannot be tolerated.

An object of this invention is to provide a novel process for decontaminating (hydrocarbylthio)aromatic amines.

Another object is to provide such a process which is simple, economical, and capable of removing Lewis acid contaminants without the use of water.

These and other objects are attained by intimately mixing at least about two molar proportions of a solid alkali metal hydroxide with a solution of one molar proportion of a Lewis acid in a (hydrocarbylthio)aromatic amine and filtering the solids from the mixture.

(Hydrocarbylthio)aromatic amines that can be decontaminated in the practice of the invention are all of the (hydrocarbylthio)aromatic amines that may be prepared by the process of Ranken et al. These are Lewis acid-contaminated aromatic compounds bearing one or more hydrocarbylthio substituents on a carbocyclic or heterocyclic ring such as a benzene, naphthalene, pyrrole, pyridine, or indole ring which has an amino nitrogen in the ring and/or bears one or more amino groups on the ring and which may bear additional substituents, such as chloro, fluoro, alkyl, alkoxy, aryl, aryloxy, alkaryl, or aralkyl substituents,

Thus, the amines include the mono- and polyhydrocarbylthio compounds prepared by reacting a hydrocarbyl disulfide, such as methyl, ethyl, propyl, n-butyl, sec-butyl, t-butyl, 2-chloropentyl, cyclopentyl, cyclohexyl, phenyl, benzyl, p-tolyl, or p-chlorophenyl disulfide with an aromatic amine such as 4,4'-methylenedianiline, 1,3-dimethylpyrrole, 1-methylpyrrole, 2-aminobiphenyl, 4-phenoxyaniline, 7-methylindole, aniline, 4-butylaniline, 4-methylaniline, 4-chloroaniline, 2-ethylaniline, N-methylaniline, 1,5-diaminonaphthalene, 2,6-diaminopyridine, 1,2-, 1,3,- and 1,4- diaminobenzenes, 2,4- and 2,6-diaminotoluenes and 2,6-diamino-1-ethylbenzene in the presence of a Lewis acid, such as a boron, aluminum, ferrous, ferric, cuprous, cupric, zinc, cadmium, lead, cobaltous, mercurous, or mercuric chloride, bromide, or iodide, a reactive metal such as aluminum, or a metal alkyl such as triethylaluminum or diethylaluminum chloride.

In a preferred embodiment of the invention, the solution that is treated is a solution of a boron or metal halide Lewis acid in one or more mono- or polyhydro-carbylthio-substituted aromatic diamines, especially such diamines wherein the hydrocarbylthio groups are alkylthio groups containing 1-6 carbons. This solution is usually the reaction product resulting from a Ranken-type process, although a solution obtained by adding a Lewis acid to a preformed (hydrocarbylthio)aromatic amine can also be successfully treated.

The alkali metal hydroxide may be lithium, sodium, potassium, rubidium, or cesium hydroxide but is preferably sodium hydroxide. To permit intimate admixture it is used in particulate form such as a powder, flakes, or granules; and it is conveniently employed as a bed of particles over which the contaminated amine can flow. The amount of hydroxide employed should be such as to provide at least about two, preferably at least about four, hydroxyl groups for each metal atom or equivalent in the Lewis acid. There does not appear to be any maximum to the amount that may be used except for the maximum that may be set by economic considerations.

In the decontamination process, the solid alkali metal hydroxide is intimately mixed with the Lewis acid/amine solution at any suitable temperature, generally at ambient temperature or at a higher temperature up to about 120° C. and preferably in the range of 110-120° C., until the Lewis acid concentration has been reduced to an acceptable level. The time required varies with the particular hydroxide and temperature used and with the ultimate Lewis acid concentration sought but is typically in the range of 1-8 hours.

The invention is advantageous as a simple, economical method of removing a Lewis acid contaminant from a (hydrocarbylthio)aromatic amine without the use of water. The effectiveness of the solid alkali metal hydroxide in the process is surprising, since the hydroxide is not soluble in the amine, and other hydroxides, such a calcium hydroxide, are relatively ineffective when used in solid form.

The following examples are given to illustrate the invention and are not intended as a limitation thereof.

EXAMPLE I

A solution of about 3 mol % of cuprous iodide in about 93 mol % of di(methylthio) derivatives, about 3 mol% of mono(methylthio) derivatives, and about 1 mol % of tri(methylthio) derivatives was prepared by reacting a commercial toluenediamine containing 80% 2,4-diaminotoluene and 20% 2,6-diaminotoluene with methyl disulfide in the presence of cuprous iodide. After excess methyl disulfide was stripped from the reaction mixture, solid sodium hydroxide was mixed with the solution to provide a mixture containing four mols of sodium hydroxide per mol of cuprous iodide, and the mixture was heated at 110-120° C. for eight hours. Samples were taken periodically, filtered, and analyzed to determine the amount of copper remaining in the amine.

| Time (hrs.) | Wt. % Cu |
|---|---|
| 0 | 1.3 |
| 1 | 0.06 |
| 2 | 0.05 |
| 4 | 0.04 |
| 6 | 0.04 |
| 8 | 0.04 |

EXAMPLE II

Example I was repeated except that the amount of sodium hydroxide mixed with the crude amine was six mols per mol of cuprous iodide. Analyses showed that the amount of copper in the product was reduced to 0.04 wt. % in only one hour and remained at that level during subsequent heating.

EXAMPLE III

Example I was repeated except that the amount of sodium hydroxide mixed with the crude amine was only three mols per mol of cuprous iodide and heating was maintained for only two hours. After two hours the amount of copper in the product was reduced to 0.2 wt. %.

EXAMPLE IV

Example I was repeated except that the sodium hydroxide was replaced with potassium hydroxide. The results of the analyses are shown below.

| Time (hrs.) | Wt. % Cu |
|---|---|
| 0 | 1.3 |
| 1 | 0.1 |
| 2 | 0.2 |
| 4 | 0.07 |
| 6 | 0.07 |
| 8 | 0.08 |

EXAMPLE V

Example I was essentially repeated except that the crude amine was one that had been prepared in the presence of a mixture of cuprous iodide and boron trifluoride etherate. The results of the analyses are shown below.

| Time (hrs.) | Wt. % Cu | Wt. % B |
|---|---|---|
| 0 | 0.67 | 0.012 |
| 1 | 0.17 | 0.009 |
| 2 | 0.14 | 0.006 |
| 4 | 0.16 | 0.004 |
| 6 | 0.14 | 0.003 |
| 8 | 0.12 | 0.001 |

## Claims

1. A process which comprises separating a Lewis acid from a (hydrocarbylthio)aromatic amine by intimately mixing at least about two molar proportions of a solid alkali metal hydroxide with a solution of one molar proportion of a Lewis acid in a (hydrocarbylthio)aromatic amine and filtering the solids from the mixture.

2. A process as claimed in claim 1 in which the alkali metal hydroxide is sodium hydroxide.

3. A process as claimed in claims I or 2 in which the Lewis acid is a boron or metal halide or mixture thereof.

4. A process as claimed in claim 1 in which the Lewis acid is cuprous iodide.

5. A process as claimed in claim 1 in which the (hydrocarbylthio)aromatic amine comprises at least one aromatic diamine bearing one or more alkylthio groups containing 1-6 carbons.

6. A process as claimed in claim 1 in which the (hydrocarbylthio)aromatic amine is a mixture of methylthio-substituted toluenediamines.

7. A process as claimed in claim 1 in which at least about four molar proportions of alkali metal hydroxide are mixed with the solution.

8. A process as claimed in claims 1, 4 or 5 in which the mixture is maintained at 110-120°C. until the Lewis acid concentration in the solution has been reduced.

9. A process as Claimed in claim 1 in which at least about four molar proportions of solid sodium hydroxide are mixed with a solution of one molar proportion of a metal halide Lewis acid in a mixture of methylthio-substituted toluenediamines, the mixture is heated at 110-120°C., and the solids are then filtered from the mixture.

## Patentansprüche

1. Verfahren zur Abtrennung einer Lewis-Säure von einem (Hydrocarbylthio)-aromatischen Amin durch inniges Vermischen von mindestens etwa zwei Molteilen eines festen Alkalimetallhydroxids mit einer Lösung von einem Molteil einer Lewis-Säure in einem (Hydrocarbylthio)-aromatischen Amin und Abfiltrieren der Feststoffe aus der Mischung.

2. Verfahren nach Anspruch 1, bei dem das Alkalimetallhydrodxid Natriumhydroxid ist.

3. Verfahren nach Anspruch 1 oder 2, bei dem die Lewis-Säure ein Bor- oder Metallhalogenid oder deren Mischung ist.

4. Verfahren nach Anspruch 1, bei dem die Lewis-Säure Kupferiodid ist.

5. Verfahren nach Anspruch 1, bei dem das (Hydrocarbylthio)-aromatische Amin mindestens ein aromatisches Diamin umfaßt, das eine oder mehrere Alkylthiogruppen mit 1 bis 6 Kohlenstoffatomen aufweist.

6. Verfahren nach Anspruch 1, bei dem das (Hydrocarbylthio) aromatische Amin eine Mischung von methylthiosubstituierten Toluoldiaminen ist.

7. Verfahren nach Anspruch 1, bei dem mindestens etwa 4 Molteile des Alkalimetallhydroxids mit der Lösung gemischt werden.

4

8. Verfahren nach Anspruch 1, 4 oder 5, bei dem die Mischung bei 110°C bis 120°C gehalten wird bis sich die Konzentration der Lewis-Säure in der Lösung vermindert hat.

9. Verfahren nach Anspruch 1, bei dem mindestens etwa vier Molteile von festem Natriumhydroxid mit einer Lösung von einem Molteil eines Lewis-Säure-Metallhalogenids in einer Mischung von methylthiosubstituierten Toluoldiaminen gemischt werden, die Mischung auf 100°C bis 120°C erhitzt wird und anschließend die Feststoffe von der Mischung abfiltriert werden.

## Revendications

1. Procédé consistant à séparer un acide de Lewis d'une amine (hydrocarbylthio)aromatique en mélangeant intimement au moins environ 2 proportions molaires d'une hydroxyde de métal alcalin solide avec une solution d'une proportion molaire d'un acide de Lewis dans une amine (hydrocarbylthio) aromatique et en séparant les solides par filtration du mélange.

2. Procédé selon la revendication 1, caractérisé en ce que l'hydroxyde de métal alcalin est de l'hydroxyde de sodium.

3. Procédé selon la revendication 1 ou 2, caractérisé en ce que l'acide de Lewis est un halogénure de bore ou de métal ou un mélange de ceux-ci.

4. Procédé selon la revendication 1, caractérisé en ce que l'acide de Lewis est un iodure cupreux.

5. Procédé selon la revendication 1, caractérisé en ce que l'amine (hydrocarbylthio) aromatique comprend au moins une diamine aromatique portant un ou plusieurs groupes alkylthio contenant 1 à 6 atomes de carbone.

6. Procédé selon la revendication 1, caractérisé en ce que l'amine (hydrocarbylthio) aromatique est un mélange de toluènediamines méthylthio-substituées.

7. Procédé selon la revendication 1, caractérisé en ce qu'au moins environ 4 proportions molaires d'hydroxyde de métal alcalin sont mélangées à la solution.

8. Procédé selon la revendication 1, 4 ou 5, caractérisé en ce que le mélange est maintenu à 110–120 °C jusqu'à ce que la concentration en acide de Lewis dans la solution ait été réduite.

9. Procédé selon la revendication 1, caractérisé en ce qu'au moins 4 proportions molaires d'hydroxyde de sodium solide sont mélangées à une solution d'une proportion molaire d'un acide de Lewis d'halogénure de métal dans un mélange de toluènediamines méthylthio-substituées, le mélange étant chauffé à 110–120°C et les solides étant ensuite extraits du mélange par filtration.